# EUROPEAN PATENT SPECIFICATION

(11) **EP 1 119 366 B1**
(45) Date of publication and mention of the grant of the patent: **26.03.2003**
(21) Application number: 99949158.2
(22) Date of filing: 06.10.1999
(51) Int. Cl.: A61K 35/78, A23K 1/18, A61K 31/44, A61K 31/34, A61K 31/415, A61K 33/10, A61K 33/42, A61P 1/04, A61P 25/00

(54) **ANIMAL STEREOTYPY**
VERHALTENSSTÖRUNGEN BEIM TIER
STEREOTYPIE CHEZ L'ANIMAL

(30) Priority: 06.10.1998 GB 9821790; 15.10.1998 GB 9822563
(43) Date of publication of application: 01.08.2001
(73) Proprietor: MARS UK LIMITED, Slough, Berkshire SL1 4LW (GB)
(72) Inventor: NICOL, Christine Div. Animal Health & Husbandry, Langford North Somerset BS40 5DU (GB); HARRIS, Patricia, Hartest Suffolk IP29 4NA (GB)
(74) Representative: Davies, Jonathan Mark
(86) International application number: GB9903288
(87) International publication number: WO00020016

(56) References cited:
- EP-A- 0 797 929
- WO-A-96/20709
- WO-A-96/31213
- WO-A-96/31239
- WO-A-98/43644
- GB-A- 1 097 955
- GB-A- 2 200 027
- L.C. WINSKILL ET AL.: "The effect of a foraging device (a modified 'Edinburgh Foodball') on the behaviour of the stabled horse" APPLIED ANIMAL BEHAVIOUR SCIENCE, vol. 48, no. 1-2, 1996, pages 25-35, XP002094083
- K.G. JOHNSON ET AL.: "Behavioural changes in stabled horses given nontherapeutic levels of virginiamycin" EQUINE VETERINARY JOURNAL, vol. 30, no. 2, 1998, pages 139-143, XP002094082 GB cited in the application
- J.G. WILLARD ET AL.: "Effect of diet on cecal pH and feeding behavior of horses" JOURNAL OF ANIMAL SCIENCE, vol. 54, no. 1, 1977, pages 87-92, XP002094085 US cited in the application
- I. REDBO ET AL.: "Factors affecting behavioural disturbances in race-horses" ANIMAL SCIENCE, vol. 66, no. 2, 1998, pages 475-481, XP002094084 GB
- L. WINSKILL ET AL.: "Stereotypies in the stabled horse : Causes, treatments and prevention" CURRENT SCIENCE, vol. 69, no. 4, 1995, pages 310-316, XP002094086
- P. MCGREEVY ET AL.: "Physiological and behavioral consequences associated with short-term prevention of crib-biting in horses" PHYSIOLOGY & BEHAVIOR, vol. 65, no. 1, 1998, pages 15-23, XP002094087 GB
- WALTER W.H. HORSEKEEPERS ENCL. 1988, pages 71 - 72
- BAYLEY L. ET AL UNDERSTANDING YOUR HORSE 1997, pages 66 - 67
- MCBANE S. BEHAVIOUR PROBLEMS IN HORSES 1995, pages 173 - 175
- HAMILTON E.S. THE STABLE MANUAL AND HORSE DOCTOR pages 349 - 351
- MAYHEW E. THE ILLU. HORSE DOCTOR 1988, pages 168 - 171

## Description

The present invention describes the treatment, prevention or amelioration of animal stereotypies.

Stereotypies are animal behavioural disorders. They are characterised by the performance of repetitive, invariant movements which have no obvious function. Equine stereotypies are of particular concern to owners of horses because the condition and performance of a horse which displays stereotypic behaviour is often adversely affected. This can substantially reduce the market value of a horse. Equine stereotypies include oral stereotypies such as crib-biting, wood chewing and wind-sucking, and locomotor stereotypies such as weaving and box-walking.

The cause or causes of stereotypies are not known. This lack of knowledge has severely hampered the development of effective treatments and preventatives for stereotypies. In the abstract of a study by Christine Nicol and Amanda Waters, entitled "The treatment and Prevention of Equine Stereotypies", theories on possible causes of equine stereotypy are given. It is noted that stereotypies are frequently regarded as functionless pathologies of the nervous system. It has also been proposed that oral stereotypies serve some digestive function. Alternative views are that they are developed by an animal as a way of dealing with stress or boredom. A further theory is that animals learn to perform stereotypies by imitating other animals that perform them.

Preventative measures for equine stereotypy based on these theories include use of stable toys to stop a horse from becoming bored or stressed, or isolation of a horse from other horses to stop it from learning stereotypies by imitation. Treatment of oral stereotypies such as crib-biting can involve more harsh measures, for example fitting the horse with a collar to prevent it from crib-biting, or even surgery. Typically, surgery involves cutting the ventral neck muscles and/or the nerves that supply them. Other forms of control include aversion therapy. Here, the horse may be given an electric shock, or physical admonishment when it performs a stereotypy.

The above treatments or preventative measures have been found to be unsatisfactory. Use of stable toys has not been found to be an effective way of preventing equine stereotypy. Physical prevention of stereotypy, either by use of a collar or surgery, is not successful because the animal still has the urge to perform the behaviour. When the collar is removed, a horse will often perform a stereotypy more intensively than before. After surgery, the animal may still be able to perform the stereotypy by utilising other muscle groups. Preventatives such as social isolation, collar fitting, aversion therapy and surgery are undesirable.

In the abstract by Nicol and Waters referred to above, it is disclosed that an epidemiological study has shown that a significant number of horses develop stereotypic behaviour during the immediate post-weaning period. At weaning, the mare-foal bond is broken, but feeding and housing practices are often also changed at this time. The discovery that stereotypic behaviour often begins in the immediate post-weaning period has not so far led to a treatment or preventative for stereotypy because it is not clear which factor or combination of factors are significant in the onset of stereotypy.

Some studies have reported a link between behavioural abnormalities and acidity in the hindgut. Johnson *et al* (Equine Veterinary Journal 1998, 30(2), 139-43) noted a reduction in abnormal behaviour when horses were administered Founderguard (containing 1% virginiamycin). The most obvious explanation for this is stated to be reduced acidosis in the hindgut caused by suppression by virginiamycin of lactic acid production in the hindgut.

Willard *et al* (Journal of Animal Science 1977, 54(1), 87-92) discloses that horses fed a concentrate diet with hourly infusions of sodium carbonate were observed to spend less time chewing wood and performing coprophagy (eating faeces) than horses fed a concentrate diet alone. The infusions of sodium carbonate significantly increased caecal pH. It was concluded that increased caecal acidity may influence the horse's desire to practice coprophagy and wood chewing.

The link between acidity in the hind gut and abnormal behaviour is also reported in WO 96/20709. This document discloses that starch, sugar or other carbohydrate which enters the hind gut is rapidly fermented to form lactic acid. This accumulation of lactic acid is stated to lead to a decline in hindgut pH which results in a wide range of biological consequences, including behavioural abnormalities. Methods of treatment or prophylaxis of adverse behaviour are disclosed in which an effective amount of an agent capable of preventing or controlling fermentative acidosis in the hindgut is administered to an animal.

Agents disclosed in WO 96/20709 as being capable of preventing or controlling fermentative acidosis are: antibiotic type compounds such as Virginiamycin (stated to be active against bacteria which produce lactic acid); enzymes which increase the digestion of carbohydrate and decrease the amount of rapidly fermentable carbohydrate passed to the hindgut; and clay preparations which bind specific ions to reduce the adverse effects of rapid fermentation of starch and other soluble carbohydrates in the gastrointestinal tract.

However, there are disadvantages to use of these agents. Virginiamycin is believed to have growth promoting activity. Consequently, this side effect may make administration of Virginiamycin undesirable. The efficacy of enzymes which increase the digestion of carbohydrate is thought to be low because the rate of passage of enzymes through the gut can be rapid and their activity may be reduced by the low pH at certain points in the gut. It is likely that high levels of clay preparation are required to be effective in reducing hind gut pH. It may be undesirable to feed an animal the levels of clay that are required to have an effect.

It is also believed that hindgut acidity is not the principal cause of at least some stereotypies. Consequently, treatments which reduce hindgut acidity may not be wholly effective and may not have any effect at all on some stereotypies.

There is, therefore, still an urgent need to provide effective treatments, preventatives and amelioratives for stereotypy which do not involve any undesirable practices being performed on an animal being treated.

We have now appreciated that there is a link between low stomach pH and behavioural abnormality. This link has not previously been recognised.

According to the invention there is provided use of a composition which comprises fat, fibre, and optionally a stomach antacid, in the manufacture of a medicament for the treatment, prevention or amelioration of animal stereotypy, wherein the amount of fat in the composition is from 5% to 20% by weight of the composition, and the amount of fibre in the composition is from 3.5% to 35% crude fibre, or from 15% to 70% neutral detergent fibre by weight of the composition.

The composition may be together with a pharmaceutically acceptable carrier, excipient or diluent to form a pharmaceutical composition.

Preferably the amount of fat is from 8% to 17% by weight of the composition. Preferred fats are highly polyunsaturated vegetable oils. These fats tend to be highly palatable and easily mixed into other components of compositions of the invention. Examples are corn oil, soya oil, or processed canola oil. Other preferred fats are more saturated fats which are more stable and, therefore, are less prone to rancidity. Examples are coconut oil, palm oil, or sunflower oil.

"Fibre" as used herein means carbohydrate which is not digestible by mammalian enzymes. Some of the fibre may be fermentable by microbial enzymes. The amount of fibre in compositions according to the invention'should be sufficient to extend the amount of time spent chewing the composition by an animal administered with such a composition. The amount of fibre in a composition of the invention may be measured as the amount of crude fibre or neutral detergent fibre (NDF).

Preferably the amount of crude fibre is from 10% to 25% by weight of the composition. The percentage of crude fibre in a sample is the percentage of the matter remaining in the sample after treatment with acid and alkali. This is measured by treating the defatted sample successively with boiling solutions of sulphuric acid and sodium hydroxide. The residue is filtered, washed, weighed and ashed. The loss of weight on ashing corresponds to the weight of fibre present in the test sample. The amount of NDF in compositions according to the invention is preferably from 25% to 50% by weight of the composition. This can be calculated using a method such as that described in Agric. Handbook No. 379 (1970) Goering H.H. and Van Soest P.T. (USDA Washington D.C.).

Preferably at least some of the fibre is chopped fibre. Preferably the chopped fibre is 1-7cm long. Preferably at least some of the fibre also has a high protein concentration. A preferred example is alfalfa hay.

The starch content of compositions according to the invention is preferably below 20% by weight of the composition. Feed stuffs which comprise high amounts of fat and fibre and low amounts of starch are especially preferred as components of compositions of the invention. An example is rice bran.

Controlling the stomach pH of an animal can treat, prevent, or ameliorate animal stereotypy.

There is provided according to the invention use of a composition comprising a stomach antacid which controls the stomach pH of an animal in the manufacture of a medicament for the treatment, prevention, or amelioration of animal stereotypy.

The stomach pH of the animal may be controlled by administering a composition according to the invention to the animal.

Administering a composition according to the invention to an animal may treat, prevent, or ameliorate animal stereotypy by preventing or reducing ulcer formation in the stomach of the animal or by treating ulcers formed in the stomach of the animal.

Suitable stomach antacids for use in compositions of the invention may act by neutralising stomach acid or by inhibiting secretion of acid into the stomach. Any alkali which neutralises stomach acid and can be safely administered to an animal may be used. Suitable antacids which act by neutralising stomach acid include Neigh-Lox and prostaglandin analogues. The active ingredients in Neigh-Lox are dihydro-aluminium sodium carbonate and aluminium phosphate. Suitable antacids which inhibit acid secretion in the stomach include proton pump inhibitors and histamine type-2 antagonists which block histamine-stimulated gastric acid secretion. Substituted benzimidazoles, such as omeprazole, act as proton pump inhibitors. Cimetidine and ranitidine are examples of Histamine type-2 antagonists.

It is believed that once an animal has learnt a stereotypy, the stereotypic behaviour becomes fixed and the animal will perform the stereotypy even if the original cause of the behaviour has been removed. Consequently, the animal should be treated using a composition according to the invention before any stereoptypic behaviour becomes fixed, and preferably before, or soon after, the animal develops any stereotypic behaviour.

Acidity in the stomach is thought to increase when animals are fed meals of grain or are subjected to extended periods of fasting. Stomach pH may also decrease when the diet of an animal changes during weaning. Consequently, an animal should be treated with a composition according to the invention shortly before and/or during and/or following eating a high grain diet, undergoing a period of extended fasting, or weaning. Treatment at these times may be particularly effective in preventing, treating, or ameliorating animal stereotypy.

In order to minimise the risk of an animal developing a stereotypy, the animal may be treated with a composition according to the invention from birth.

Compositions described herein may also be effective when the animal being treated is a weaned animal.

It is considered that compositions according to the invention will usually be included with the diet of an animal.

Compositions according to the invention may be included in the diet of the animal's mother while she is lactating. This is because the mother's diet influences the nutritional content of the milk which the animal receives and because the animal may eat its mother's feed before it is weaned; foals invariably eat their dam's feed before they are weaned.

Compositions according to the invention may be included in feed and the said feed fed to the animal as it is being weaned onto solid food.

Compositions according to the invention may be included in the animal's diet post weaning.

The invention is further described by the following embodiment. The embodiment relates to use of stomach antacid to treat, ameliorate, or prevent crib biting in horses.

### Example

### Methods

Advertisements were placed for foals to take part in the study. Foals offered for the study were rejected if the foal had been crib-biting for more than 20 weeks, was more than 1 year of age, or if the owner had attempted to prevent crib-biting using surgery or electric shock treatments.

Foals chosen for the study were visited at times when the owners had previously noted crib-biting behaviour. They were observed for a minimum of 1 hour to establish that they were performing crib-biting behaviour. It was clear from the observations that some owners had mistaken wood-chewing for crib-biting.

A crib-biting horse grasps a fixed object with its incisor teeth, arches its neck, and pulls back (often but not always emitting a grunting sound). There is no ingestion of wood, or other substrate. The behaviour is invariant in form - it tends to occur in the same place, or perhaps 2 or 3 favoured sites, within the stable, at the same time in a sequence of behaviour (for example a horse may grasp a piece of hay, move to the front of its stable, then crib-bite), and at the same times of day (for example just after feeding). The crucial part of crib-biting is arching of the neck which puts considerable tension on the neck muscles, and affects the oesophagus and pharynx.

Wood chewing is completely different and cannot be distinguished in form from normal chewing at hay, straw or bark. Wood or other material is ingested and chewed. The wood or material may or may not be swallowed. The horse is not tense during this behaviour. A horse will perform wood-chewing in a variety of places (wherever a new bit of wood can be found), and does not perform the behaviour as part of a fixed sequence of behaviour.

Those foals observed to be wood-chewing were rejected from further study. The remaining foals were randomly allocated to a feed treatment, and their owners were provided with an initial supply of the diet for that treatment and asked to gradually change their foals onto this diet over the subsequent week.

In total, 13 crib-biting foals and 8 control foals (belonging to 13 different owners) were recruited. Comparisons were made between the two populations and are summarised in Table 1.

**Table 1**

| **General Characteristics of Study Population** | | | | |
|---|---|---|---|---|
| | **Crib-biting foals** | | **Normal foals** | |
| | mean | se | mean | se |
| Age at entry (days)+ | 233.7 | 9.21 | 291.2 | 47.45 |
| Age at weaning (days) | 172.38 | 9.00 | 183.62 | 15.20 |
| Age when concentrate introduced (days) | 40.0 | 18.1 | 51.2 | 29.80 |
| Amount of concentrate fed prior to entry (kg) | 2.64 | 0.30 | 2.61 | 0.40 |

| | | | | |
|---|---|---|---|---|
| + Entry into the study was taken as the first date on which detailed behaviour observations were taken | | | | |

Crib-biting foals had developed crib-biting behaviour at an average of 152.5 (se 20.3) days of age, or 21.8 weeks. A proportion of the foals had developed oral stereotypy prior to weaning. They had been performing stereotypic behaviour for a mean 88.7 (se 13.3) days, or 12.7 weeks, prior to entry into the study.

### Treatments

Six crib-biting and four control foals were allocated to a typical base diet containing cereals (wheat, barley, oats), wheatfeed, soya bean meal, peas, full fat linseed, vitamin and mineral supplements, and molasses. This base diet was supplemented with forage (fresh or preserved). This is Diet A. This diet was re-bagged before delivery to the owners and was fed according to body weight.

Seven crib-biting and three control foals were allocated to an antacid diet. This was Diet A, re-bagged and supplied with a tub of Neigh-Lox. Owners were asked to feed approximately 125g of Neigh-Lox per day, divided equally among feeds.

One control foal for which initial samples and endoscopy results were obtained was not placed on a diet as its crib-biting partner moved to a different yard.

### Behavioural Observations

Participants were visited regularly throughout the trial. Owners were asked to ensure that their foal was in an environment where it had been observed crib-biting for at least one hour before the behaviour recordings were started. The time of day, and time in relation to meal-time, that observations were taken varied among foals, but was generally held constant for each foal in the study across repeated visits.

### Endoscopy

Foals were endoscoped during the first week of the trial, and a week after the trial had ended. All foals were deprived of all feed and forage for a period of 12 hours overnight and endoscoped between 0900 and 1030 hours the next morning. During endoscopy. A continuous video record was made of the glandular and squamous mucosa. Samples of gastric fluid were taken and the pH measured. The veterinarian performing the endoscopy then provided a written description of his observations. An independent observer who was not given information about which horses were crib-biters, or about which feed treatment they had received also prepared a descriptive account from the video recordings. This was compared and the written description prepared at the time by the veterinarian. The information was summarised according to an agreed scoring system.

### RESULTS

### Baseline correlations among variables and differences between crib-biting and normal foals at the start of the trial.

### (i) General Characteristics

None of the normal foals started to crib-bite during the trial.

There were no significant differences between crib-biting and normal foals in any of the general population parameters, although despite very small numbers, there was a trend towards an association between crib-biting and previous administration of antibiotics.

### (ii) Endoscopy

Endoscopy records were obtained for 10 crib-biting and 4 control horses at the start of the trial.

Ulcers were observed in 6 foals at the start of the trial. The ulcers were few in number and generally mild, with the exception of 1 crib-biting foal that had extensive ulceration. The crib-biting foals had higher scores for number of bots, severity of ulcers and inflammation than the normal foals at the start of the trial, and lower scores for stickiness of the mucosa, black flecks on the mucosa, corrugations of the squamous mucosa and folding of the glandular mucosa. The squamous mucosa and glandular mucosa were also less moist in crib-biting than normal foals.

The mean pH of the gastric fluid samples taken from 5 crib-biting foals on the first endoscopy visit was 1.69 with a range between 1.43 and 2.00.

### Treatment Effects

### (i) Behaviour

The data on frequency and duration of crib-biting is shown in Tables 2 and 3. It should be noted that the management of some of the foals changed during the course of the study. These foals were No. 1: in all the time (observations 1 and 2) to out all the time (observation 3); No. 3: in at night or in all of the time if wet (observations 1 and 2) to out all of the time (observation 3); and No. 13: hay fed as forage (observations 1 and 2) to haylage fed as forage (observation 3). The third observation period for these 3 foals was therefore not used in analysis.

**Table 2**

| **The frequency of crib-biting (mean bites per hour) exhibited during the trial** | | | | |
|---|---|---|---|---|
| **Foal** | **Diet** | **Obs period 1** | **Obs period 2** | **Obs period 3** |
| 1 | Control | 187.3 | 85.5 | (3.8) |
| 2 | Control | 5.5 | 0.0 | 0.0 |
| 3 | Control | 220.0 | 449.0 | (225.0) |
| 4 | Control | 48.4 | 2.5 | 0 |
| 5 | Control | 11.5 | 0.5 | 3.25 |
| 6 | Control | 62.7 | 70.0 | 73.5 |
| 7 | Neighlox | 4.5 | 0.5 | 0.0 |
| 8 | Neighlox | 60.5 | 478.5 | 155 |
| 9 | Neighlox | 285.3 | 204.9 | 223 |
| 10 | Neighlox | 111.5 | 47.7 | Deceased |
| 11 | Neighlox | 30.0 | 16.3 | 9.00 |
| 12 | Neighlox | 13.5 | 20.5 | 13.9 |
| 13 | Neighlox | 41.25 | 16.75 | (100) |

**Table 3 -**

| **The duration of crib-biting (mean seconds per hour) exhibited during trial** | | | | |
|---|---|---|---|---|
| **Foal** | **Diet** | **Obs period 1** | **Obs period 2** | **Obs period 3** |
| 1 | Control | 894.5 | 821.5 | (22) |
| 2 | Control | 41.5 | 0.0 | 0.0 |
| 3 | Control | 976.0 | 2326.0 | (691.0) |
| 4 | Control | 278 | 23.3 | 0 |
| 5 | Control | 148 | 8.3 | 18.0 |
| 6 | Control | 599.7 | 574.5 | 674 |
| 7 | Neighlox | 89.2 | 5.0 | 0.0 |
| 8 | Neighlox | 364.8 | 2328.5 | 1007.0 |
| 9 | Neighlox | 1767.0 | 945 | 1278 |
| 10 | Neighlox | 455.5 | 172.2 | Deceased |

The change in crib-biting frequency and duration was assessed by taking the slope of the values over the 3 month period. Four of the 13 crib-biting foals had positive slope values for both frequency and duration, indicating an overall increase in crib-biting behaviour during the trial. The remaining slopes were all negative.

Each foal was then ranked according to its change in behaviour over time, relative to the other foals in the study. The two measures of frequency and duration resulted in slightly different rank orderings of the foals. For both measures rank order 1 indicates the foal that showed the largest decline in crib-biting, and rank order 13 indicates the foal that showed the largest increase in crib-biting. The rankings are shown in Table 4 and were used to examine associations between stomach condition and behavioural change.

**Table 4**

| **Rank ordering of foals according to change in cribbiting behaviour during trial** | | |
|---|---|---|
| **Foal** | **Rank Order for frequency** | **Rank order for duration** |
| 1 | 1 | 5 |
| 2 | 8 | 9 |
| 3 | 13 | 13 |
| 4 | 5 | 3 |
| 5 | 7 | 6 |
| 6 | 11 | 11 |
| 7 | 9 | 7 |
| 8 | 12 | 12 |
| 9 | 3 | 2 |
| 10 | 2 | 1 |
| 11 | 6 | 4 |
| 12 | 10 | 10 |
| 13 | 4 | 8 |

### (ii) Endoscopy

Valid endoscopy records were obtained for 8 crib-biters and 3 normal horses. Positive correlations were obtained between the presence of ulcers and the presence of bots, between the presence of bots and inflammation of the mucosai surface, and between the presence of ulcers and inflammation of the mucosal surface.

The crib-biting foals had higher scores for number of bots, number of ulcers, and inflammation than the normal foals, and lower scores for stickiness of the mucosa, corrugations of the squamous mucosa and foling of the glandular mucosa, results that were very similar to the starting conditions. However, in contrast to the start of the trial the squamous mucosa and glandular mucosa were moister in crib-biting than normal foals.

Data from crib-biting and normal horses were combined and subjected to analysis to examine the effects of dietary treatment on stomach condition. At the end of the trial, horses that had received Neigh-Lox had fewer ulcers and less inflammation than horses that had received the control diets. Eight horses had noticeable ulcers at the start of the trial. Those with mild ulcers that were fed Neighlox all resolved by the end of the trial. Those with moderate or severe ulcers that were fed Neigh-Lox either showed no improvement or got worse. The ulcers of horses that were fed the control diet showed no change or got worse; none of these ulcers resolved spontaneously.

The relationship between ulcers and crib-biting behaviour was examined by comparing the extent to which crib-biting changed over the course of the trial, with the severity of ulcers present at the end of the trial. Horses whose ulcers did not heal during the course of the trial were also the horses that showed little or no reduction in crib-biting behaviour.

The results presented here demonstrate for the first time a relationship between stomach condition and abnormal oral behaviour in the horse and are consistent with crib-biting being an adaptive attempt to reduce stomach acidity. Crib-biting foals tended to have more bots and ulcers, a drier and more expanded stomach wall, and a greater degree of inflammation than normal foals. The general appearance of the stomach of the crib-biting foals supports the hypothesis that their stomachs are more acid. The results also show that an improvement in stomach condition was associated with reduced crib-biting behaviour. Administration of Neigh-Lox was associated with a resolution of mild ulceration. Foals whose mild ulceration cleared showed the greatest improvement in crib-biting. In some of these foals crib-biting ceased altogether.

The cause of the stomach problems in crib-biting foals is not clear. Candidate factors include:
- The early introduction of concentrate feed.
   Most of the crib-biting foals in the study had received concentrate feed from birth, or during the pre-weaning period. It is known from other work that concentrate feed increases gastric acidity, and causes ulceration.
- Previous illness or use of antibiotics.
   The owners of some foals reported that they had started crib-biting during a period of illness or after receiving antibiotics. Illness may involve confinement and separation and foals may not feed properly during such periods.
   Alternatively, antibiotics may have a more direct effect in disrupting the flora of the hindgut.
- Sustained effects originating at weaning.
   Weaning by methods that are particularly stressful increases the rate of development of steriotypies dramatically. Stressed foals are unlikely to eat, and feed deprivation is known to increase gastric acidity.
- Differential production of saliva.
   It is possible that there is variation among foals in the extent to which they release saliva, either spontaneously or during feeding. The production of a limited supply of saliva may cause or enhance stomach acidity. The foals that do the most crib-biting may be the ones that most need to produce saliva and are most frustrated by their inability to do so in sufficient quantities.

The data presented in the example demonstrate the effectiveness of a stomach antacid on the prevention, treatment, or amelioration of *equine* crib-biting. This activity is thought to be enhanced by the inclusion in compositions according to the invention of fat and fibre.

Compositions according to the invention may be particularly effective at preventing stereotypy when the animal being treated is a weaning or recently weaned animal. Foals are typically weaned when they are four to six months old.

Use of compositions according to the invention may be particularly effective in the amelioration, treatment or prevention of any stereotypy in all *equidae*, non-ruminant herbivores, and non-ruminant omnivores, for example crib-biting, wind-sucking, weaving and box-walking in equine animals.

Use of compositions according to the invention may be particularly effective in the amelioration, treatment or prevention of stereotypies linked with gut function in all *equidae*, non-ruminant herbivores, and non-ruminant omnivores, but especially in the amelioration, treatment or prevention of equine crib-biting.

The reason that compositions according to the invention may be particularly effective in the amelioration, treatment or prevention of animal stereotypy is not known. However, the realisation that low stomach pH is linked with stereotypic behaviour suggests that pain caused by low stomach pH may cause the animal to perform a stereotypy, such as crib-biting, to stimulate the flow of saliva into the stomach. This saliva would be expected to increase the stomach pH and alleviate the pain. The fact that significant numbers of horses develop stereotypic behaviour during the immediate post-weaning period may be because the diet of a foal changes significantly during weaning. If such a dietary change results in a persistent decrease in stomach pH, then stereotypic behaviour may be more likely to occur. Administration of compositions according to the invention to an animal, especially a weaning or recently weaned animal, may ensure that its stomach pH is not persistently low and remove, therefore, the need for the animal to stimulate the flow of saliva into the stomach. It is believed that the fibre may help to prolong the time spent chewing by an animal. This in turn prolongs the production of saliva which neutralises stomach acid. The fat is thought to delay emptying of the stomach so that the beneficial effect of the antacid and/or fibre is prolonged.

Compositions according to the invention may act by preventing or reducing stomach ulcer formation caused by prolonged periods of low stomach pH, or by treating stomach ulcers already formed.

Compositions according to the invention may be significantly more effective in the treatment and prevention of animal stereotypy than prior treatments and preventatives. In addition, treatment of animals using compositions according to the invention does not involve any undesirable practices being performed on the animal.

## Claims

1. Use of a composition comprising fat, fibre, and optionally a stomach antacid, in the manufacture of a medicament for the amelioration, treatment, or prevention of animal stereotypy, wherein the amount of fat in the composition is from 5% to 20% by weight of the composition, and the amount of fibre in the composition is from 3.5% to 35% crude fibre, or from 15% to 70% neutral detergent fibre, by weight of the composition.

2. Use according to claim 1, wherein the composition is a pharmaceutical composition and is together with a pharmaceutically acceptable carrier, excipient or diluent.

3. Use according to claim 1 or 2 in which at least some of the fibre is chopped fibre.

4. Use according to claim 3 in which at least some of the chopped fibre is about 1-7cm long.

5. Use according to any preceding claim in which the starch content of the composition is below 20% by weight of the composition.

6. Use according to any preceding claim in which the antacid inhibits secretion of acid in the stomach.

7. Use according to claim 6 in which the antacid is a proton pump inhibitor such as omeprazole, or a histamine type-2 antagonist.

8. Use of a composition comprising a stomach antacid which controls the stomach pH of an animal in the manufacture of a medicament for the amelioration, treatment, or prevention of animal stereotypy.

9. Use according to claim 8, wherein the stomach antacid comprises dihydro-aluminium sodium carbonate and aluminium phosphate, a prostaglandin analogue, or an antacid which inhibits acid secretion in the stomach.

10. Use according to any preceding claim in which the animal is an *equidae*, a non-ruminant omnivore, or a non-ruminant herbivore.

11. Use according to claim 10 in which the animal is a horse.

12. Use according to claim 11 in which the stereotypy is crib-biting.

## Patentansprüche

1. Verwendung einer Zusammensetzung, die Fett, Faser und bei Bedarf ein Magen-Antazidum umfasst, zur Herstellung eines Medikamentes zur Linderung, Behandlung oder Vorbeugung einer Tierstereotypie, wobei die Fettmenge in der Zusammensetzung 5 bis 20 Gew.-% der Zusammensetzung ausmacht und die Fasermenge der Zusammensetzung 3,5 bis 35 Gew.-% Rohfaser oder 15 bis 70 Gew.-% Neutraldetergensfaser der Zusammensetzung ausmacht.

2. Verwendung nach Anspruch 1, wobei die Zusammensetzung eine pharmazeutische Zusammensetzung ist, zusammen mit einem pharmazeutisch akzeptablen Träger, Bindemittel oder Verdünnungsmittel.

3. Verwendung nach Anspruch 1 oder 2, wobei wenigstens ein Teil der Faser zerhackte Faser ist.

4. Verwendung nach Anspruch 3, wobei wenigstens ein Teil der zerhackten Faser etwa 1-7 cm lang ist.

5. Verwendung nach einem der vorherigen Ansprüche, wobei der Stärkegehalt der Zusammensetzung unter 20 Gew.-% der Zusammensetzung liegt.

6. Verwendung nach einem der vorherigen Ansprüche, wobei das Antazidum eine Sekretion von Säure im Magen hemmt.

7. Verwendung nach Anspruch 6, wobei das Antazidum ein Protonenpumpeninhibitor wie Omeprazol oder ein Histamin-Typ-2-Antagonist ist.

8. Verwendung einer Zusammensetzung, die ein Magen-Antazidum umfasst, das den pH-Wert des Magens eines Tieres kontrolliert, zur Herstellung eines Medikamentes zur Linderung, Behandlung oder Vorbeugung einer Tierstereotypie.

9. Verwendung nach Anspruch 8, wobei das Magen-Antazidum Dihydro-Aluminium-Natriumcarbonat und Aluminiumphosphat, ein Prostaglandin-Analog oder ein Antazidum umfasst, das Säuresekretion im Magen hemmt.

10. Verwendung nach einem der vorherigen Ansprüche, wobei das Tier ein Equidae, ein nicht wiederkäuender Omnivore oder ein nicht wiederkäuender Herbivore ist.

11. Verwendung nach Anspruch 10, wobei das Tier ein Pferd ist.

12. Verwendung nach Anspruch 11, wobei die Stereotypie Koppen ist.

## Revendications

1. Utilisation d'une composition comprenant de la matière grasse, de la fibre et facultativement un antiacide gastrique, dans la fabrication d'un médicament pour l'amélioration, le traitement ou la prévention d'une stéréotypie animale, où la quantité de matière grasse dans la composition est de 5% à 20% en poids de la composition, et la quantité de fibre dans la composition est de 3,5% à 35% de fibre brute, ou de 15% à 70% de fibre neutre détergente, en poids dans la composition.

2. Utilisation selon la revendication 1, où la composition est une composition pharmaceutique et est avec un véhicule, un excipient ou un diluant pharmaceutiquement acceptable.

3. Utilisation selon la revendication 1 ou 2, où une certaine partie de la fibre au moins est de la fibre coupée.

4. Utilisation selon la revendication 3, où une certaine partie de la fibre coupée fait environ 1-7 cm de long.

5. Utilisation selon l'une quelconque des revendications précédentes, où la teneur en amidon de la composition est inférieure à 20% en poids de la composition.

6. Utilisation selon l'une quelconque des revendications précédentes, où l'antiacide inhibe une sécrétion acide dans l'estomac.

7. Utilisation selon la revendication 6, où l'antiacide est un inhibiteur de la pompe à protons tel que l'oméprazole, ou un antagoniste de l'histamine de type 2.

8. Utilisation d'une composition comprenant un antiacide gastrique qui contrôle le pH de l'estomac d'un animal, dans la fabrication d'un médicament pour l'amélioration, le traitement ou la prévention d'une stéréotypie animale.

9. Utilisation selon la revendication 8, où l'antiacide gastrique comprend du carbonate de sodium-dihydroaluminium et du phosphate d'aluminium, un analogue de la prostaglandine ou un antiacide qui inhibe une sécrétion acide dans l'estomac.

10. Utilisation selon l'une quelconque des revendications précédentes, où l'animal est un équidé, un omnivore non ruminant ou un herbivore non ruminant.

11. Utilisation selon la revendication 10, où l'animal est un cheval.

12. Utilisation selon la revendication 11, où la stéréotypie est le tic.
